# EUROPEAN PATENT SPECIFICATION

(11) **EP 1 572 269 B1**
(45) Date of publication and mention of the grant of the patent: **04.06.2008**
(21) Application number: 03753744.6
(22) Date of filing: 01.10.2003
(51) Int. Cl.: A61M 5/32

(54) **IMPROVEMENTS IN OR RELATING TO HYPODERMIC SYRINGES**
VERBESSERUNGEN BEZÜGLICH SUBKUTANINJEKTIONSSPRITZEN
AMELIORATIONS APPORTEES A UNE SERINGUE HYPODERMIQUE

(30) Priority: 01.10.2002 GB 0222731
(43) Date of publication of application: 14.09.2005
(73) Proprietor: Parker, David William, Bury, Lancashire BL8 4QQ (GB); Burgess, Colin Hamilton, Ramsbottom, Lancashire BL0 9QR (GB)
(72) Inventor: Parker, David William, Bury, Lancashire BL8 4QQ (GB); Burgess, Colin Hamilton, Ramsbottom, Lancashire BL0 9QR (GB)
(74) Representative: Draper, Martyn John
(86) International application number: PCT/GB2003/004326
(87) International publication number: WO 2004/030732

(56) References cited:
- EP-A- 1 092 442
- WO-A-00/18454
- WO-A-01/72362
- US-A- 4 955 870
- US-A- 5 330 430

## Description

This invention relates to hypodermic syringes where a needle assembly may be retracted into a syringe housing after use. In particular, the invention relates to a retractable hypodermic syringe suitable for the delivery of small doses of injectant.

It is now well-known that there is a worldwide need for safe hypodermic syringes, the most effective of these being a passive syringe with a retracting needle. Examples of such syringes are disclosed in our co-pending patent applications WO00/18454, WO01/43619 and WO01/72362. These earlier applications disclose syringes in which, at the completion of an injection stroke, a plunger becomes attached to a needle carrier. A stored energy mechanism, such as a spring, is then triggered to retract the needle carrier and needle into the syringe housing.

However, the provision of a needle retraction mechanism in a small capacity syringe is not straightforward. The reliability of a needle retraction mechanism can only be ensured if adequate stored energy is provided to overcome friction between components of the syringe and any resistance from a bent needle. Prior syringes have comprised stored energy configurations that are limited by the dimensions of the syringe, i.e. based on the cross-sectional area of the injectant chamber. The dimensions of the injectant chamber of a small capacity syringe typically range from around 6 mm in diameter, giving a cross sectional area of 28 mm², for a 1 ml. syringe to 10 mm diameter, i.e. a cross sectional area of 78 mm², for a 3 ml. syringe. making the provision of a suitable stored energy means problematical.

An object of the present invention is to provide a retraction facility that is particularly suitable for syringes of small capacity, i.e. 3 ml or less.

In a first aspect of the present invention, a hypodermic syringe comprises a housing, said housing including a barrel portion and an injectant chamber portion, the injectant chamber portion being of a smaller cross-sectional area than the barrel portion, a plunger slideably mounted within the barrel portion, comprising a piston which extends into the injectant chamber portion, a retractable needle assembly and a stored energy means, configured so that, at the completion of an injection stroke, the piston becomes attached to the needle assembly and the stored energy in the stored energy means is released to retract the needle assembly into the housing. The stored energy means may be located in the plunger.

The syringe may be arranged so that, following needle retraction, residual stored energy is used to retain the needle and plunger within the housing.

The syringe may comprise non-reversible snap-fitting formations for securely retaining the needle assembly within the syringe after use.

The needle assembly may comprise a first part that has a non-circular cross-section and is located in a non-circular aperture of the housing, wherein the first part and the aperture co-operate to prevent rotation of the needle assembly relative to the housing.

The injectant chamber portion may have a capacity of 3 ml or less.

The stored energy means may be a spring.

The needle assembly may be mounted in the housing, during assembly of the syringe, with the needle pre-sheathed.

The needle assembly may comprise a seal in order to reduce friction during needle retraction.

One or more components of the syringe may be located and retained by the use of snap-fits during assembly.

The syringe may comprise a plunger closure piece, said plunger closure piece having an aperture.

The invention will now be described by way of an example with reference to the accompanying drawing.

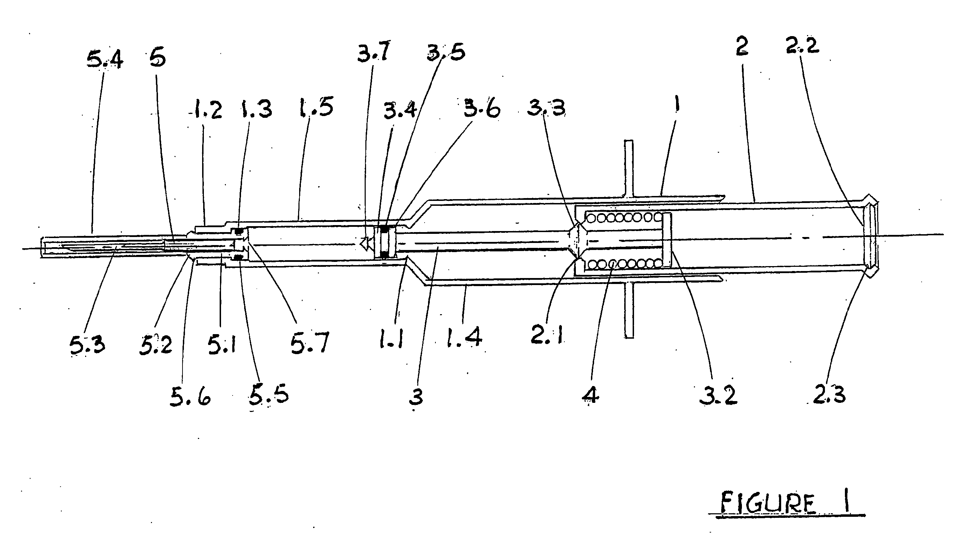

Figure 1 depicts a syringe according to the present invention, comprising a body 1 with a proximal end and a distal end 1.2, including a barrel 1.4, a constriction 1.1, a portion of reduced diameter 1.3 and an injectant chamber 1.5. An assembly including a plunger 2, a piston rod 3 and a spring 4 is slideably mounted within the body 1.

The body 1 houses a needle assembly 5, which comprises of a two part needle carrier 5.1, 5.2 and a seal 5.5 and is mounted in the body 1 by a snap-fit. A sheath 5.4 is also provided, which covers a needle 5.3 mounted on the needle assembly 5. The first part 5.1 of the needle assembly 5 has a non-circular cross-section and is located in a non-circular aperture in the distal end 1.2 of the body 1, in order to prevent rotation of the needle 5.3 relative to the body 1. This facilitates subsequent removal of the sheath 5.4 so that the sheath 5.4 can be removed using a twisting motion, e.g. by gripping the sheath 5.4 and rotating the body 1 without causing such a rotation.

The needle assembly 5, needle 5.3 and sheath 5.4 are dimensioned to pass through the constriction 1.1 in the body 1. This allows positioning of the needle assembly 5 within the body 1 during assembly of the syringe with the needle 5.3 pre-sheathed. The needle assembly 5, needle 5.3 and sheath 5.4 are inserted through the proximal end of the body 1 and snap into a position in the distal end 1.2 of the body 1. In this position, the seal 5.5 is located in a reduced diameter portion 1.3 of the injectant chamber 1.5. The needle carrier part 5.2 includes a feature 5.6, e.g. a button or ridge, which provides a reversible retaining catch against the body 1.

The piston 3 is fitted with the spring 4 bearing against a flange 3.2 and is mounted through the proximal end of the plunger 2. The spring 4 is compressed until a reversible catch formed by a collar 3.3 and an orifice 2.1 is engaged to retain the spring 4 under compression.

The assembly thus formed of plunger 2 and piston 3 is inserted into the barrel 1.4 of the body 1. When the piston 3 meets the constriction 1.1, a flange 3.4 and a seal 3.5 located on the piston 3 will pass through with little resistance, but a second flange 3.6 is so dimensioned that it snaps through the restriction 1.1, with which it interacts to form a virtually non-reversible catch. A plunger closure piece 2.2, which may have a central hole for assembly purposes, is snap-fitted into a recess 2.3 formed at the proximal end of the plunger 2.

Operation of the syringe follows closely established practice. Following removal of the sheath 5.4, the plunger 2 is pressed in order to cause air in the injectant chamber 1.5 to be expelled, but not so far as to engage a non-reversible catch formed by a socket 5.7 located in the needle assembly 5 and a barb 3.7 on the piston 3. The injectant is drawn in the usual way and any necessary adjustments are made to expel air and to ensure that the correct quantity of injectant has been loaded.

Pressure is applied to the plunger 2 in the usual way to effect injection, expelling the injectant. At the end of an injection stroke, an end face of the piston 3 abuts the needle carrier 5 and the two interacting features, i.e. the socket 5.7 and the barb 3.7 engage.

Further pressure on the plunger 2 will cause reversal of the catch formed by the collar 3.3 of the piston 3 and the orifice 2.1 of the plunger 2, releasing the stored energy of the spring 4 so that the needle assembly 5 is retracted. The design ensures that a spring 4 of sufficient strength can be used to overcome the friction of the piston seal 3.5 and the reversible catch formed by the feature 5.6 against the distal end of the body 1. Once released, the needle assembly 5 and needle 5.3, both being of lesser diameter than the injectant chamber 1.5, do not resist retraction.

The needle retraction movement is halted by the piston flange 3.6 meeting the constriction 1.1 in the body 1. The remaining spring energy draws the plunger 2 into the body 1 so that the plunger closure piece 2.2 is flush with the proximal end of the body 1 and the whole assembly is locked in a secure position.

In addition to providing a reliable needle retraction mechanism, the embodiment also has the following attributes:
- Accurate metering;
   In order to achieve this, good visibility of the piston 3 and injectant chamber 1.5 is maintained, as the components of the needle retraction mechanism do not obscure the user's view of the injectant chamber 1.5. Furthermore, as the injectant chamber 1.5 has a small cross-section, the length of the injectant chamber 1.5 is sufficient to allow clear calibration spacing;
- Complete evacuation of the injectant chamber;
   Complete evacuation is desirable for reasons of injectant cost or aggressiveness. This attribute is provided by maximising the contact between a needle piston 3 and the body 1, in particular the contact between the piston flange 3.4 and the distal end 1.2 of the body. This allows the piston 3 to sweep the total length of the injectant chamber 1.5 so that the evacuation of the injectant is not impeded by any components of the needle release mechanism;
- Security after use for safe disposal;
   The needle is locked in a retracted position after use without requiring any additional action by the user, i.e. in a completely automatic process forming part of the needle retraction sequence;
- Instinctive operation following established principles;
   The syringe maintains the feel of a standard syringe in both handling and operation, thereby reducing both the incidence of errors and the need for special training of its users.
- Low cost and simple assembly;
   Low unit cost is achieved as the syringe contains a reduced the number of components, when compared with standard retractable syringes. In addition, the components are of forms that can be simply moulded. The simple assembly process described above is also a significant contributor to low cost.

The embodiment described comprises a needle which is fitted during the manufacturing process, as is usual for syringes of small capacity. However, if a need arises for needle replacement, or for the fitting a needle to the syringe by the user, the syringe may allow the threading of part of a needle between the first and second parts 5.1, 5.2 of the needle assembly. Similarly, while the injectant chamber 1.5 of the embodiment is cylindrical, i.e. with a circular cross-section, it is not necessary for the injectant chamber to have this particular shape.

## Claims

1. A hypodermic syringe comprising:
a housing (1), said housing (1) including a barrel portion (1.4) and an injectant chamber portion (1.5);
a plunger (2) slideably mounted within the barrel portion (1.4), comprising a piston (3) which extends into the injectant chamber portion (1.5);
a retractable needle assembly (5); and
a stored energy means (4);
configured so that, at the completion of an injection stroke, the piston (3) becomes attached to the needle assembly (5) and the stored energy in the stored energy means (4) is released to retract the needle assembly (5) into the housing (1);
**characterised by:**
the injectant chamber portion (1.5) having a smaller cross-sectional area than the barrel portion (1.4).

2. A hypodermic syringe according to claim 1, wherein the stored energy means (4) is located in the plunger (2).

3. A hypodermic syringe according to claim 1 or 2, wherein, following needle retraction, residual stored energy is used to retain the needle (5.3) and plunger (2) within the housing (1).

4. A hypodermic syringe according to claim 1, 2 or 3, further comprising non-reversible snap-fitting formations for securely retaining the needle assembly (5) within the syringe after use.

5. A hypodermic syringe according to any one of the preceding claims, wherein a first part (5.1) of the needle assembly (5) has a non-circular cross-section and is located in a non-circular aperture of the housing (1), wherein the first part (5.1) and the aperture co-operate to prevent rotation of the needle assembly (5.3) relative to the housing (1).

6. A hypodermic syringe according to any one of the preceding claims, wherein the injectant chamber portion (1.5) has a capacity of 3 ml or less.

7. A hypodermic syringe according to any of the preceding claims, wherein the stored energy means (4) is a spring.

8. A hypodermic syringe according to any one of the preceding claims, wherein, during assembly of the syringe, the needle assembly (5) is mounted in the housing (1) with the needle (5.3) pre-sheathed.

9. A hypodermic syringe according to any one of the preceding claims, wherein the needle assembly (5) comprises a seal (5.5) in order to reduce friction during needle retraction.

10. A hypodermic syringe according to one of the preceding claims in which one or more components of the syringe are located and retained by the use of snap-fits during assembly.

11. A hypodermic syringe according to any one of the preceding claims, further comprising a plunger closure piece (2.2), said plunger closure piece (2.2) having an aperture.

## Patentansprüche

1. Subkutane Spritze, umfassend:
ein Gehäuse (1), wobei das Gehäuse (1) einen Rohrabschnitt (1.4) und einen Injektionsmittel-Kammerabschnitt (1.5) enthält;
einen Stößel (2), der innerhalb des Rohrabschnitts (1.4) verschiebbar angebracht ist und einen Kolben (3) aufweist, der sich in den Injektionsmittel-Kammerabschnitt (1.5) hinein erstreckt;
eine einfahrbare Nadelanordnung (5); und
ein Energiespeichermittel (4);
derart konfiguriert, dass bei Abschluss des Injektionshubs der Kolben (3) an der Nadelanordnung (5) angebracht wird und die in dem Energiespeichermittel (4) gespeicherte Energie freigegeben wird, um die Nadelanordnung (5) in das Gehäuse (1) einzufahren;
**dadurch gekennzeichnet, dass** der Injektionsmittel-Kammerabschnitt (1.5) eine kleinere Querschnittsfläche als der Rohrabschnitt (1.4) hat.

2. Subkutane Spritze nach Anspruch 1, worin das Energiespeichermittel (4) in dem Stößel (2) angeordnet ist.

3. Subkutane Spritze nach Anspruch 1 oder 2, worin, nach dem Einfahren der Nadel, gespeicherte Restenergie benutzt wird, um die Nadel (5.3) und den Stößel (2) in dem Gehäuse (1) zurückzuhalten.

4. Subkutane Spritze nach Anspruch 1, 2 oder 3, die ferner nicht reversible Schnappsitzausformungen aufweist, um nach Gebrauch die Nadelanordnung (5) innerhalb der Spritze sicher zurückzuhalten.

5. Subkutane Spritze nach einem der vorhergehenden Ansprüche, worin ein erstes Teil (5.1) der Nadelanordnung (5) einen nicht kreisförmigen Querschnitt hat und in einer nicht kreisförmigen Öffnung des Gehäuses (4) angeordnet ist, wobei das erste Teil (5.1) und die Öffnung zusammenwirken, um eine Drehung der Nadelanordnung (5.3) relativ zu dem Gehäuse (1) zu verhindern.

6. Subkutane Spritze nach einem der vorhergehenden Ansprüche, worin der Injektionsmittel-Kammerabschnitt (1.5) eine Kapazität von 3 ml oder weniger hat.

7. Subkutane Spritze nach einem der vorhergehenden Ansprüche, worin das Energiespeichermittel (4) eine Feder ist.

8. Subkutane Spritze nach einem der vorhergehenden Ansprüche, worin, während des Zusammenbaus der Spritze, die Nadelanordnung (5) mit vorab ummantelter Nadel (5.3) in dem Gehäuse (1) angebracht wird.

9. Subkutane Spritze nach einem der vorhergehenden Ansprüche, worin die Nadelanordnung (5) eine Dichtung (5.5) aufweist, um während des Einfahrens der Nadel Reibung zu reduzieren.

10. Subkutane Spritze nach einem der vorhergehenden Ansprüche, worin eine oder mehrere Komponenten der Spritze während des Zusammenbaus durch die Verwendung von Schnappsitzen positioniert und gehalten werden.

11. Subkutane Spritze nach einem der vorhergehenden Ansprüche, die ferner ein Stößelverschlusstück (2.2) aufweist, wobei das Stößelverschlusstück (2.2) eine Öffnung hat.

## Revendications

1. Seringue hypodermique comportant :
un corps (1), ledit corps (1) comprenant une partie cylindrique (1.4) et une partie (1.5) de chambre de produit d'injection ;
un plongeur (2) monté de façon coulissante dans la partie cylindrique (1.4), comportant un piston (3) qui pénètre dans la partie de chambre (1.5) de produit d'injection ;
un ensemble à aiguille rétractable (5) ; et
un moyen (4) à énergie emmagasinée ;
configurée de manière que, à la fin d'une course d'injection, le piston (3) se trouve attaché à l'ensemble à aiguille (5) et l'énergie emmagasinée dans le moyen (4) à énergie emmagasinée est libérée pour rétracter l'ensemble à aiguille (5) dans le corps (1) ;
**caractérisée en ce que** :
la partie (1.5) de chambre de produit d'injection a une aire en section transversale inférieure à celle de la partie cylindrique (1.4).

2. Seringue hypodermique selon la revendication 1, dans laquelle le moyen à énergie emmagasinée (4) est placé dans le plongeur (2).

3. Seringue hypodermique selon la revendication 1 ou 2, dans laquelle, à la suite du retrait de l'aiguille, de l'énergie emmagasinée résiduelle est utilisée pour retenir l'aiguille (5.3) et le plongeur (2) à l'intérieur du corps (1).

4. Seringue hypodermique selon la revendication 1, 2 ou 3, portant en outre des formations d'encliquetage non réversibles destinées à retenir solidement l'ensemble à aiguille (5) à l'intérieur de la seringue après l'utilisation.

5. Seringue hypodermique selon l'une quelconque des revendications précédentes, dans laquelle une première partie (5.1) de l'ensemble à aiguille (5) a une section transversale non circulaire et est placé dans une ouverture non circulaire du corps (1), la première partie (5.1) et l'ouverture coopérant de façon à empêcher l'ensemble à aiguille (5.3) de tourner par rapport au corps (1).

6. Seringue hypodermique selon l'une quelconque des revendications précédentes, dans laquelle la partie de chambre (1.5) à produit d'injection a une capacité de 3 ml ou moins.

7. Seringue hypodermique selon l'une quelconque des revendications précédentes, dans laquelle le moyen (4) à énergie emmagasinée est un ressort.

8. Seringue hypodermique selon l'une quelconque des revendications précédentes, dans laquelle, pendant l'assemblage de la seringue, l'ensemble à aiguille (5) est monté dans le corps (1) avec l'aiguille (5.3) préalablement gainée.

9. Seringue hypodermique selon l'une quelconque des revendications précédentes, dans laquelle l'ensemble à aiguille (5) comporte un joint d'étanchéité (5.5) pour réduire le frottement pendant le retrait de l'aiguille.

10. Seringue hypodermique selon l'une quelconque des revendications précédentes, dans laquelle un ou plusieurs constituants de la seringue sont placés et retenus par l'utilisation d'encliquetages pendant l'assemblage.

11. Seringue hypodermique selon l'une quelconque des revendications précédentes, comportant en outre une pièce (2.2) de fermeture du plongeur, ladite pièce (2.2) de fermeture du plongeur ayant une ouverture.
